# EUROPÄISCHE PATENTSCHRIFT

(11) **EP 4 085 869 B1**
(45) Veröffentlichungstag und Bekanntmachung des Hinweises auf die Patenterteilung: **16.08.2023**
(21) Anmeldenummer: 22167978.0
(22) Anmeldetag: 12.04.2022
(51) Int. Cl.: A61F 2/16, B29D 11/02, B29D 11/00, G02B 3/14

(54) **VERFAHREN ZUM HERSTELLEN EINER AKKOMMODATIVEN INTRAOKULARLINSE**
METHOD OF MANUFACTURING AN ACCOMMODATING INTRAOCULAR LENS
PROCÉDÉ DE FABRICATION D'UNE LENTILLE INTRAOCULAIRE À ACCOMMODATION

(30) Priorität: 27.04.2021 DE 102021110782
(43) Veröffentlichungstag der Anmeldung: 09.11.2022
(73) Patentinhaber: Carl Zeiss Meditec AG, 07745 Jena (DE)
(72) Erfinder: SCHREIBER, Benjamin, 10551 Berlin (DE)
(74) Vertreter: PATERIS Patentanwälte PartmbB

(56) Entgegenhaltungen:
- EP-A2- 0 435 525
- WO-A1-2011/137191
- US-A- 5 185 107
- US-A1- 2016 058 553

## Beschreibung

Die Erfindung betrifft ein Verfahren zum Herstellen einer akkommodativen Intraokularlinse.

Unter Akkommodation wird die Fähigkeit des Auges verstanden, die Brechkraft dynamisch anzupassen, und dadurch Gegenstände in unterschiedlicher Entfernung scharf zu sehen. Dabei wird eine Durchmesseränderung eines Ziliarmuskels des Auges über Zonulafasern auf einen flexiblen Kapselsack übertragen, in dem eine flexible Linse angeordnet ist. Allerdings verschlechtert sich mit zunehmendem Alter die Fähigkeit zu der Akkommodation, was zur Folge eine Altersweitsichtigkeit hat. Dies liegt vor allem in einer geringer werdenden Elastizität der Linse, die sich mit dem zunehmenden Alter verfestigt. Im Rahmen der Behandlung eines Katarakts, auch grauer Star genannt, wird die Linse durch eine künstliche Intraokularlinse ersetzt. Die Intraokularlinse kann beispielsweise monofokal sein, mehrere Fokusse aufweisen oder einen erweiterten Fokusbereich aufweisen. Es existieren jedoch auch Konzepte, bei denen die Intraokularlinse akkommodativ ausgeführt ist und somit die Möglichkeit besteht, die akkommodative Fähigkeit des Auges wiederherzustellen. Die akkommodative Intraokularlinse weist zum Beispiel eine verformbare Membran auf, die einen mit einem Fluid gefüllten Hohlraum in dem Inneren der Intraokularlinse begrenzt. Beim Verformen des Kapselsacks werden die Membran und der Hohlraum verformt, wodurch eine Verlagerung des Fokuses der Intraokularlinse erfolgt und somit die Brechkraft wie bei der natürlichen Linse dynamisch angepasst werden kann.

Herkömmlich wird die akkommodative Intraokularlinse hergestellt, indem ein kleiner Schnitt in die Intraokularlinse eingebracht wird, das Fluid via den Schnitt in den Hohlraum injiziert wird und anschließend der Schnitt wieder versiegelt wird. Dies ist jedoch ein aufwändiges Verfahren.

EP 0 435 525 A2 offenbart eine multifokale ophthalmische Linse. US 2016/0030161 A1 offenbart eine akkommodierende Intraokularlinse. WO 2011/137191 offenbart ein Verfahren zum Herstellen einer akkommodativen Intraokularlinse.

Aufgabe der Erfindung ist es daher, ein Verfahren zum Herstellen einer akkommodativen Intraokularlinse zu schaffen, wobei das Verfahren einfach durchführbar ist.

Das erfindungsgemäße Verfahren zum Herstellen einer akkommodativen Intraokularlinse weist die Schritte auf: a) Bereitstellen eines ersten Bauteils, eines zweiten Bauteils und eines Stützkörpers, der einen Innenraum aufweist und nach oben offen ist, so dass der Innenraum von oben zugänglich ist; b) Befestigen des ersten Bauteils an dem Stützkörper, so dass der Innenraum von dem ersten Bauteil begrenzt ist und das erste Bauteil einen oberhalb des ersten Bauteils angeordneten Außenraum von dem Innenraum trennt; c) Erzeugen eines höheren Drucks in dem Außenraum als in dem Innenraum, so dass sich das erste Bauteil nach unten verformt; d) Erzeugen einer Klebefläche an der oberen Seite des ersten Bauteils und/oder an dem zweiten Bauteil; e) Aufbringen einer Flüssigkeit von oben auf das erste Bauteil in dessen nach unten verformten Bereich, wobei zu keinem Zeitpunkt die Flüssigkeit die Klebefläche kontaktiert; f) Befestigen des zweiten Bauteils an dem ersten Bauteil mittels der Klebefläche, wodurch die akkommodative Intraokularlinse gebildet wird und die Flüssigkeit in einem im Inneren der Intraokularlinse angeordneten Hohlraum von dem ersten Bauteil und dem zweiten Bauteil eingekapselt ist.

Wenn die Flüssigkeit die Klebefläche kontaktieren würde, wäre es nicht möglich, das zweite Bauteil an dem ersten Bauteil zu befestigen. Indem in Schritt c) das erste Bauteil nach unten verformt wird, ist es möglich, zu allen Zeitpunkten die Flüssigkeit fern von der Klebefläche zu halten. Durch das erfindungsgemäße Verfahren ist es nicht erforderlich, zum Herstellen der akkommodativen Intraokularlinse einen Schnitt in die akkommodative Intraokularlinse einzubringen, der hinterher wieder aufwändig versiegelt werden müsste. Damit ist das erfindungsgemäße Verfahren zum Herstellen der akkommodativen Intraokularlinse einfach durchführbar.

Es ist bevorzugt, dass das Verfahren den Schritt aufweist: g) Erzeugen eines gleichen Drucks in dem Innenraum wie in dem Außenraum. Dadurch kann die akkommodative Intraokularlinse leicht von dem Stützkörper entfernt werden. Dabei ist auch denkbar, dass das Verfahren den Schritt aufweist: h) Erzeugen eines höheren Drucks in dem Innenraum als in dem Außenraum. Dadurch kann die akkommodative Intraokularlinse noch leichter von dem Stützkörper entfernt werden oder entfernt sich von alleine von dem Stützkörper.

Es ist bevorzugt, dass das erste Bauteil einen Optikkörper aufweist und das zweite Bauteil eine Membran aufweist. Alternativ ist es bevorzugt, dass das erste Bauteil eine Membran aufweist und das zweite Bauteil einen Optikkörper aufweist. Alternativ ist es bevorzugt, dass das erste Bauteil und das zweite Bauteil jeweils eine Membran aufweisen. Die Membran ist vorformbar und ein Verformen der Membran führt auch zu einem Verformen des mit der Flüssigkeit gefüllten Hohlraums. Dadurch kann der Fokus der akkommodativen Intraokularlinse besonders einfach verlagert werden.

Die Membran ist bevorzugt gasdurchlässig. In dem Fall, dass zusammen mit der Flüssigkeit auch etwas Gas aus dem Außenraum in dem Hohlraum angeordnet ist, kann das Gas besonders leicht durch die Membran nach außerhalb der akkommodativen Intraokularlinse gelangen.

Es ist bevorzugt, dass das in dem Außenraum angeordnete Gas ein Edelgas, insbesondere Helium und/oder Argon, mit einem Volumenanteil von mindestens 80 % aufweist. Besonders bevorzugt beträgt der Volumenanteil des Heliums mindestens 90 %, mindestens 95 % oder mindestens 99 %. In dem Fall, dass zusammen mit der Flüssigkeit auch etwas Gas aus dem Außenraum in dem Hohlraum angeordnet ist, kann das Helium via das erste Bauteil und/oder das zweite Bauteil besonders einfach nach außerhalb der akkommodativen Intraokularlinse gelangen.

In Schritt d) wird die Klebefläche bevorzugt durch ein Aufbringen eines Klebers erzeugt. Bei dem Kleber kann es sich beispielsweise um einen Silikonkleber handeln. Alternativ ist es bevorzugt, dass in Schritt d) die Klebefläche durch ein Durchführen einer Oberflächenbehandlung erzeugt wird. Die Oberflächenbehandlung weist besonders bevorzugt eine Behandlung mit einem Plasma auf. Beispielsweise kann das Plasma ionisierten Sauerstoff aufweisen.

In Schritt f) wird der Stützkörper bevorzugt erwärmt. Dadurch kann ein Aushärten des Klebers beschleunigt werden oder das zweite Bauteil kann schneller mittels der durch die Oberflächenbehandlung erzeugten Klebefläche an dem ersten Bauteil befestigt werden.

Der Schritt d) wird bevorzugt vor dem Schritt e) durchgeführt. Alternativ ist es bevorzugt, dass der Schritt e) vor dem Schritt d) durchgeführt wird.

Die Flüssigkeit hat bevorzugt im Bereich des sichtbaren Lichts einen höheren Brechungsindex als Wasser. Es ist auch denkbar, dass die Brechzahl der Flüssigkeit gleich der Brechzahl des ersten Bauteils und/oder des zweiten Bauteils ist. Dadurch können Reflektionsverluste an dem Übergang von der Flüssigkeit zu dem ersten Bauteil und/oder dem zweiten Bauteil vermieden werden.

Der Stützkörper ist bevorzugt im Wesentlichen ringförmig. Der Stützkörper kann an seinem unteren Ende offen ausgebildet sein, insbesondere wenn in Schritt c) die Atmosphäre in dem Innenraum abgesaugt wird. Alternativ ist denkbar, dass der Stützkörper bis auf sein oberes Ende geschlossen ausgebildet ist, insbesondere wenn in Schritt c) der Druck in dem Außenraum erhöht wird.

Es ist bevorzugt, dass in Schritt b) das erste Bauteil an dem oberen Ende des Stützkörpers befestigt wird.

Es ist bevorzugt, dass in Schritt a) eine Mehrzahl der Stützkörper und für jeden der Stützkörper jeweils eines der ersten Bauteile und eines der zweiten Bauteile bereitgestellt wird und in dem Verfahren eine Mehrzahl der akkommodativen Intraokularlinsen gleichzeitig hergestellt wird. Durch das gleichzeitige Herstellen der Mehrzahl der akkommodativen Intraokularlinsen ist das Verfahren kostengünstig durchführbar.

Im Folgenden wird anhand der beigefügten schematischen Zeichnungen die Erfindung näher erläutert.
Figur 1 zeigt eine erste Ausführungsform des erfindungsgemäßen Verfahrens und
Figur 2 zeigt eine zweite Ausführungsform des erfindungsgemäßen Verfahrens.

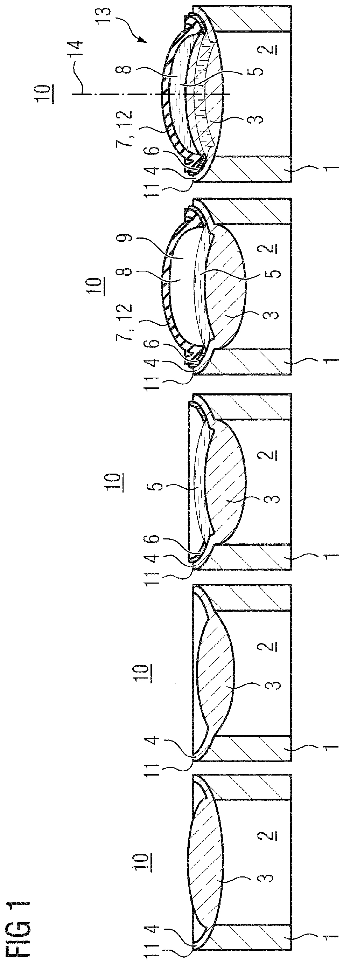

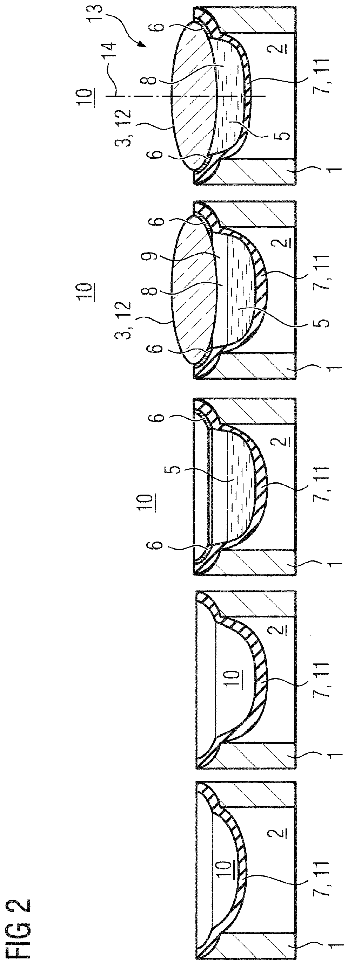

In Figuren 1 und 2 ist das Verfahren zu fünf verschiedenen Zeitpunkten dargestellt, wobei die Zeit von links nach rechts weiter fortschreitet. Das Verfahren zum Herstellen einer akkommodativen Intraokularlinse 13 weist folgende Schritte auf: a) Bereitstellen eines ersten Bauteils 11, eines zweiten Bauteils 12 und eines Stützkörpers 1, der einen Innenraum 2 aufweist und nach oben offen ist, so dass der Innenraum 2 von oben zugänglich ist; b) Befestigen des ersten Bauteils 11 an dem Stützkörper 1, so dass der Innenraum 2 von dem ersten Bauteil 11 begrenzt ist und das erste Bauteil 11 einen oberhalb des ersten Bauteils 11 angeordneten Außenraum 10 von dem Innenraum 2 trennt, siehe den jeweils ersten Zeitpunkt in Figuren 1 und 2; c) Erzeugen eines höheren Drucks in dem Außenraum 10 als in dem Innenraum 2, so dass sich das erste Bauteil 11 nach unten verformt, wie es zu dem zweiten Zeitpunkt in Figuren 1 und 2 dargestellt ist; d) Erzeugen einer Klebefläche 6 an der oberen Seite des ersten Bauteils 11, vergleiche den dritten Zeitpunkt in Figuren 1 und 2, wobei es alternativ oder zusätzlich möglich ist, die Klebefläche an dem zweiten Bauteil 12 zu erzeugen; e) Aufbringen einer Flüssigkeit 5 von oben auf das erste Bauteil 11 in dessen nach unten verformten Bereich, wobei zu keinem Zeitpunkt die Flüssigkeit 5 die Klebefläche 6 kontaktiert, siehe den dritten Zeitpunkt in Figuren 1 und 2; f) Befestigen des zweiten Bauteils 12 an dem ersten Bauteil 11 mittels der Klebefläche 6, wodurch die akkommodative Intraokularlinse 13 gebildet wird und die Flüssigkeit 5 in einem im Inneren der Intraokularlinse 13 angeordneten Hohlraum 8 von dem ersten Bauteil 11 und dem zweiten Bauteil 12 eingekapselt ist, siehe den vierten Zeitpunkt in Figuren 1 und 2. Zudem kann das Verfahren den Schritt aufweisen: Erzeugen eines gleichen Drucks in dem Innenraum 2 wie in dem Außenraum 10 nach dem Beenden des Schrittes f), vergleiche den fünften Zeitpunkt in Figuren 1 und 2.

Figuren 1 und 2 zeigen, dass in Schritt b) das erste Bauteil 11 an dem oberen Ende des Stützkörpers 1 befestigt werden kann. Das obere Ende kann mit nach innen und nach unten abfallenden Flanken ausgebildet sein, wie es in Figuren 1 und 2 dargestellt ist. Dies ist besonders vorteilhaft, wenn das erste Bauteil 11, wie es in Figuren 1 und 2 dargestellt ist, an dessen unterem Ende konvex ausgebildet ist. Dann ist das obere Ende des Stützkörpers 1 konkav ausgebildet. Alternativ ist denkbar, dass das obere Ende des Stützkörpers 1 eine nicht gewölbte, also plane Ebene aufweist, welche bevorzugt horizontal verläuft mit horizontalen Flächen ausgebildet ist.

In dem Schritt b) wird das erste Bauteil 11 so an dem Stützkörper 1 befestigt, dass kein Gasaustausch zwischen dem Innenraum 2 und dem Außenraum 10 stattfinden kann.

Indem in Schritt c) das erste Bauteil 11 nach unten verformt wird, verkleinert sich der Innenraum 2. Beispielsweise kann der Druck in dem Innenraum 2 mindestens 10 kPa weniger als in dem Außenraum 10 betragen, damit sich das erste Bauteil 11 ausreichend verformt. Beispielsweise kann der Druck in dem Innenraum 2 maximal 100 kPa weniger als in dem Außenraum 10 betragen, damit eine Beschädigung der akkommodativen Intraokularlinse 13 vermieden werden kann.

In Schritt c) ist es möglich, den Druck in dem Außenraum 10 zu erhöhen und/oder den Druck in dem Innenraum 2 zu erniedrigen. Das untere Ende des Stützkörpers 1 kann offen sein. Dadurch kann durch ein Absaugen von Atmosphäre via das untere Ende des Stützkörpers 1 der Druck in dem Innenraum 2 erniedrigt werden. Alternativ ist denkbar, dass das untere Ende des Abstützkörpers 1 geschlossen ist und dass die einzige Öffnung des Abstützkörpers 1 an dessen oberem Ende vorgesehen ist. Hier ist in Schritt c) der Druck in dem Außenraum 10 zu erhöhen. Der Stützkörper 1 kann zumindest abschnittsweise im Wesentlichen ringförmig sein unabhängig davon, ob der Stützkörper 1 an seinem unteren Ende offen oder geschlossen ausgebildet ist.

Es ist sowohl möglich, den Schritt d) vor dem Schritt e) durchzuführen, als auch den Schritt e) vor dem Schritt d) durchzuführen. In beiden Fällen ist es durch das Verformen des ersten Bauteils 11 nach unten völlig unproblematisch, die Flüssigkeit 5 von der Klebefläche 6 fernzuhalten, bis das zweite Bauteil 12 an dem ersten Bauteil 11 befestigt ist. In Schritt d) kann die Klebefläche 6 durch ein Aufbringen eines Klebers erzeugt werden. Der Kleber kann beispielsweise ein Silikonkleber sein. In einem anderen Beispiel kann in Schritt d) die Klebefläche 6 durch ein Durchführen einer Oberflächenbehandlung des ersten Bauteils 11 und/oder des zweiten Bauteils 12 erzeugt werden. Die Oberflächenbehandlung kann eine Behandlung mit einem Plasma aufweisen, wobei das Plasma beispielsweise ionisierten Sauerstoff aufweisen kann.

Die Klebefläche 6 kann beispielsweise im Wesentlichen die Form eines Kreisrings haben. Die Flüssigkeit 5 kann innerhalb des Kreisrings angeordnet sein.

Die Flüssigkeit 5 kann transparent für sichtbares Licht sein. Zudem kann die Flüssigkeit 5 eine Viskosität von maximal 2*10³ Pa*s aufweisen, wodurch eine gute Verformbarkeit der akkommodativen Intraokularlinse 13 erreicht wird. Beispielsweise kann die Flüssigkeit ein Silikonöl sein oder im Wesentlichen aus dem Silikonöl bestehen. Außerdem kann die Flüssigkeit 5 im Bereich des sichtbaren Lichts einen höheren Brechungsindex als Wasser haben.

Um das Befestigen des zweiten Bauteils 11 an dem ersten Bauteil zu beschleunigen, kann in Schritt f) der Stützkörper 1 erwärmt werden.

Gemäß der ersten Ausführungsform des Verfahrens gemäß Figur 1 weist das erste Bauteil 11 einen Optikkörper 3 auf und das zweite Bauteil 12 weist eine Membran 7 auf. Gemäß der zweiten Ausführungsform des Verfahrens gemäß Figur 2 weist das erste Bauteil 11 eine Membran 7 auf und das zweite Bauteil 12 weist einen Optikkörper 3 auf. Die Membran 7 kann gasdurchlässig sein. Dadurch wird erreicht, dass in dem Fall, dass in dem Schritt f) ein Gas 9 von dem Außenraum 10 in den Hohlraum 9 gelangt, dieses nach außerhalb der akkommodativen Intraokularlinse 13 gelangt. Das in dem Außenraum (10) angeordnete Gas 9 kann Helium mit einem Volumenanteil von mindestens 80 %, mindestens 90 % oder mindestens 99 % aufweisen. Helium ist ein Gas, was besonders schnell durch die Membran 7 nach außerhalb der akkommodativen Intraokularlinse 13 gelangt. Die Membran 7 kann eine Dicke von beispielsweise 20 µm bis 200 µm oder von 20 µm bis 150 µm haben.

Wie es aus Figur 1 ersichtlich ist, kann die akkommodative Intraokularlinse 13 eine Haptik 4 oder mehrere Haptiken 4 aufweisen, die an dem Optikkörper 3 befestigt sind. In dem Fall, dass das erste Bauteil 11 den Optikkörper 3 aufweist, sind die Haptik 4 oder die Haptiken 4 Teil des ersten Bauteils 11. In dem Fall, dass das zweite Bauteil 12 den Optikkörper 3 aufweist, sind die Haptik 4 oder die Haptiken 4 Teil des zweiten Bauteils 12. Der Werkstoff des ersten Bauteils 11 und/oder des zweiten Bauteils 12 (in Fig. 2 sind die Haptiken nicht dargestellt) kann ein Acrylpolymer aufweisen oder im Wesentlichen aus dem Acrylpolymer bestehen.

Zum Verformen der akkommodativen Intraokularlinse 13 nach einer Implantation der akkommodativen Intraokularlinse ist es denkbar, dass die akkommodative Intraokularlinse 13 eine Mehrzahl an Biegeelementen (nicht in Figuren 1 und 2 dargestellt) aufweist, die außen an der Membran 7 gleichmäßig um eine optische Achse 14 der akkommodativen Intraokularlinse 13 angeordnet sind. Ein im Wesentlichen zylinderförmiger Körper (nicht in Figuren 1 und 2 dargestellt) kann nach der Implantation der akkommodativen Intraokularlinse 13 in den Kapselsack eines Auges auf die Biegeelemente aufgesetzt werden und eine Kraft von dem Kapselsack auf die Biegeelemente übertragen, was dazu führt, dass sich die Membran 7 und der Hohlraum 8 verformen.

### Bezugszeichenliste

- 1: Stützkörper
- 2: Innenraum
- 3: Optikkörper
- 4: Haptik
- 5: Flüssigkeit
- 6: Klebefläche
- 7: Membran
- 8: Hohlraum
- 9: Gas
- 10: Außenraum
- 11: erstes Bauteil
- 12: zweites Bauteil
- 13: akkommodative Intraokularlinse
- 14: optische Achse

## Patentansprüche

1. Verfahren zum Herstellen einer akkommodativen Intraokularlinse (13) mit den Schritten:
a) Bereitstellen eines ersten Bauteils (11), eines zweiten Bauteils (12) und eines Stützkörpers (1), der einen Innenraum (2) aufweist und nach oben offen ist, so dass der Innenraum (2) von oben zugänglich ist;
b) Befestigen des ersten Bauteils (11) an dem Stützkörper (1), so dass der Innenraum (2) von dem ersten Bauteil (11) begrenzt ist und das erste Bauteil (11) einen oberhalb des ersten Bauteils (11) angeordneten Außenraum (10) von dem Innenraum (2) trennt;
c) Erzeugen eines höheren Drucks in dem Außenraum (10) als in dem Innenraum (2), so dass sich das erste Bauteil (11) nach unten verformt;
d) Erzeugen einer Klebefläche (6) an der oberen Seite des ersten Bauteils (11) und/oder an dem zweiten Bauteil (12);
e) Aufbringen einer Flüssigkeit (5) von oben auf das erste Bauteil (11) in dessen nach unten verformten Bereich, wobei zu keinem Zeitpunkt die Flüssigkeit (5) die Klebefläche (6) kontaktiert;
f) Befestigen des zweiten Bauteils (12) an dem ersten Bauteil (11) mittels der Klebefläche (6), wodurch die akkommodative Intraokularlinse (13) gebildet wird und die Flüssigkeit (5) in einem im Inneren der Intraokularlinse (13) angeordneten Hohlraum (8) von dem ersten Bauteil (11) und dem zweiten Bauteil (12) eingekapselt ist.

2. Verfahren gemäß Anspruch 1, wobei das erste Bauteil (11) einen Optikkörper (3) aufweist und das zweite Bauteil (12) eine Membran (7) aufweist oder wobei das erste Bauteil (11) eine Membran (7) aufweist und das zweite Bauteil (12) einen Optikkörper (3) aufweist.

3. Verfahren gemäß Anspruch 2, wobei die Membran (7) gasdurchlässig ist.

4. Verfahren gemäß einem der Ansprüche 1 bis 3, wobei das in dem Außenraum (10) angeordnete Gas (9) Helium mit einem Volumenanteil von mindestens 80 % aufweist.

5. Verfahren gemäß einem der Ansprüche 1 bis 4, wobei in Schritt d) die Klebefläche (6) durch ein Aufbringen eines Klebers erzeugt wird.

6. Verfahren gemäß einem der Ansprüche 1 bis 5, wobei in Schritt d) die Klebefläche (6) durch ein Durchführen einer Oberflächenbehandlung erzeugt wird, wobei die Oberflächenbehandlung insbesondere eine Behandlung mit einem Plasma aufweist.

7. Verfahren gemäß einem der Ansprüche 1 bis 6, wobei in Schritt f) der Stützkörper (1) erwärmt wird.

8. Verfahren gemäß einem der Ansprüche 1 bis 7, wobei der Schritt d) vor dem Schritt e) durchgeführt wird oder wobei der Schritt e) vor dem Schritt d) durchgeführt wird.

9. Verfahren gemäß einem der Ansprüche 1 bis 8, wobei der Stützkörper (1) ringförmig ist.

10. Verfahren gemäß einem der Ansprüche 1 bis 9, wobei in Schritt b) das erste Bauteil (11) an dem oberen Ende des Stützkörpers (1) befestigt wird.

## Claims

1. Method for producing an accommodative intraocular lens (13) including the steps of:
a) providing a first component (11), a second component (12) and a support body (1) which has an interior space (2) and is open to the top such that the interior space (2) is accessible from above;
b) fastening the first component (11) to the support body (1) such that the interior space (2) is delimited by the first component (11) and the first component (11) separates an exterior space (10) arranged above the first component (11) from the interior space (2);
c) generating a higher pressure in the exterior space (10) than in the interior space (2) so that the first component (11) deforms downward;
d) producing an adhesive surface (6) on the upper side of the first component (11) and/or on the second component (12);
e) applying a liquid (5) to the first component (11) from above into the latter's downwardly deformed region, the liquid (5) at no time contacting the adhesive surface (6) ;
f) fastening the second component (12) to the first component (11) by means of the adhesive surface (6), as a result of which the accommodative intraocular lens (13) is formed and the liquid (5) is encapsulated by way of the first component (11) and the second component (12) in a cavity (8) arranged in the interior of the intraocular lens (13).

2. Method according to Claim 1, wherein the first component (11) has an optics body (3) and the second component (12) has a membrane (7) or wherein the first component (11) has a membrane (7) and the second component (12) has an optics body (3).

3. Method according to Claim 2, wherein the membrane (7) is gas permeable.

4. Method according to any one of Claims 1 to 3, wherein the gas (9) arranged in the exterior space (10) comprises helium with a volume fraction of at least 80%.

5. Method according to any one of Claims 1 to 4, wherein the adhesive surface (6) is produced in step d) by an application of an adhesive.

6. Method according to any one of Claims 1 to 5, wherein the adhesive surface (6) is produced in step d) by carrying out a surface treatment, the surface treatment including a treatment with a plasma in particular.

7. Method according to any one of Claims 1 to 6, wherein the support body (1) is heated in step f).

8. Method according to any one of Claims 1 to 7, wherein step d) is carried out before step e) or wherein step e) is carried out before step d).

9. Method according to any one of Claims 1 to 8, wherein the support body (1) is ring shaped.

10. Method according to any one of Claims 1 to 9, wherein the first component (11) is fastened to the upper end of the support body (1) in step b).

## Revendications

1. Procédé de fabrication d'une lentille intraoculaire accommodative (13), ledit procédé comprenant les étapes suivantes :
a) fournir un premier composant (11), un deuxième composant (12) et un corps de support (1) qui comporte un espace intérieur (2) et qui est ouvert vers le haut de sorte que l'espace intérieur (2) est accessible depuis le haut ;
b) fixer le premier composant (11) au corps de support (1) de sorte que l'espace intérieur (2) soit délimité par le premier composant (11) et que le premier composant (11) sépare un espace extérieur (10), disposé au-dessus du premier composant (11), de l'espace intérieur (2) ;
c) générer une pression plus élevée dans l'espace extérieur (10) que dans l'espace intérieur (2) de sorte que le premier composant (11) se déforme vers le bas ;
d) générer une surface adhésive (6) sur la face supérieure du premier composant (11) et/ou sur le deuxième composant (12) ;
e) appliquer un liquide (5) depuis le haut sur le premier composant (11) dans sa région déformée vers le bas, le liquide (5) n'entrant à aucun moment en contact avec la surface adhésive (6) ;
f) fixer le deuxième composant (12) au premier composant (11) au moyen de la surface adhésive (6), de sorte que la lentille intraoculaire accommodative (13) est formée et le liquide (5) est encapsulé dans une cavité (8), ménagée à l'intérieur de la lentille intraoculaire (13), par le premier composant (11) et le deuxième composant (12).

2. Procédé selon la revendication 1, le premier composant (11) comportant un corps d'optique (3) et le deuxième composant (12) comportant une membrane (7) ou le premier composant (11) comportant une membrane (7) et le deuxième composant (12) comportant un corps d'optique (3).

3. Procédé selon la revendication 2, la membrane (7) étant perméable aux gaz.

4. Procédé selon l'une des revendications 1 à 3, le gaz (9) disposé dans l'espace extérieur (10) comportant de l'hélium avec une proportion en volume d'au moins 80 %.

5. Procédé selon l'une des revendications 1 à 4, à l'étape d) la surface adhésive (6) étant générée par application d'un adhésif.

6. Procédé selon l'une des revendications 1 à 5, à l'étape d) la surface adhésive (6) étant générée par un traitement de surface, le traitement de surface comportant notamment un traitement par un plasma.

7. Procédé selon l'une des revendications 1 à 6, à l'étape f) le corps de support (1) étant chauffé.

8. Procédé selon l'une des revendications 1 à 7, l'étape d) étant réalisée avant l'étape e) ou l'étape e) étant réalisée avant l'étape d).

9. Procédé selon l'une des revendications 1 à 8, le corps de support (1) étant annulaire.

10. Procédé selon l'une des revendications 1 à 9, à l'étape b) le premier composant (11) étant fixé à l'extrémité supérieure du corps de support (1).
